# EUROPEAN PATENT SPECIFICATION

(11) **EP 0 883 571 B1**
(45) Date of publication and mention of the grant of the patent: **11.07.2001**
(21) Application number: 97908785.5
(22) Date of filing: 27.02.1997
(51) Int. Cl.: C02F 1/38, C12M 1/26, C12N 1/02

(54) **CONCENTRATION OF WATERBORNE PATHOGENIC ORGANISMS**
KONZENTRIERUNG VON IN WASSERENTHALTENEN KRANKHEITSERREGERN
CONCENTRATION D'ORGANISMES PATHOGENES EN SUSPENSION DANS L'EAU

(30) Priority: 28.02.1996 US 608422
(43) Date of publication of application: 16.12.1998
(73) Proprietor: Marshfield Clinic, Marshfield, WI 54449-5777 (US)
(72) Inventor: BORCHARDT, Mark, A., Marshfield, WI 54449 (US); SPENCER, Susan, K., Spencer, WI 54479 (US)
(74) Representative: Powell, Stephen David
(86) International application number: PCT/US97/03176
(87) International publication number: WO 97/31863

(56) References cited:
- EP-A- 0 420 153
- WO-A-90/10057
- DE-A- 2 408 754
- US-A- 3 217 982
- US-A- 4 094 461
- US-A- 4 217 418
- US-A- 4 569 759
- US-A- 4 894 050
- US-A- 5 324 629

## Description

### Field of the Invention

The present invention is directed to a method of concentrating waterborne pathogenic organisms from water potentially contaminated by dilute densities of the organisms.

### Background of the Invention

Developments in water processing technology have significantly decreased the incidence of illness from waterborne pathogenic organisms. However, isolated cases of illnesses owing to such organisms are common, and occasionally large-scale outbreaks of illness occur. As an example, severe outbreaks of gastroenteritis have been caused by the environmentally resistant intestinal protozoan parasites *Cryptosporidium, Giardia, Microsporidia,* and *Cyclospora.* These organisms are often waterborne, existing in very dilute densities in public water supplies, and many have only recently been recognized as causing a health problem. As an example, *Cryptosporidium* has been linked to waterborne outbreaks of gastroenteritis only since 1981, when widespread cases of acute gastroenteritis in a Texas community were traced to the presence of *Cryptosporidium* in the drinking water supply. Numerous other cities have experienced outbreaks since then. *Cryptosporidium* oocysts are exemplary of waterborne protozoan parasites in that they can survive for long times in water and are resistant to routine water treatment methods such as chlorination. The presence in source waters of even small numbers of *Cryptosporidium* oocysts is a matter of concern because the infective dose for humans is possibly as low as one oocyst. Additionally, exposure to protozoan parasites or other waterborne pathogens can be life-threatening, particularly to infants, the elderly, and people whose immune systems are impaired. In fact, many waterborne pathogens are only now receiving significant scientific attention after having been traced to fatalities in this population, e.g., *Microsporidia.*

Clearly, the detection of pathogenic organisms such as *Cryptosporidium* in water supplies is a critical societal problem. The central issue in detection of such organisms is how to concentrate dilute densities of the organisms from large volumes of water so as to detect their presence.

Regarding protozoan parasites such as *Cryptosporidium,* the method currently recommended by the Environmental Protection Agency (EPA) and the American Society for Testing and Materials (ASTM) concentrates the parasites by sampling 100 liters (or more) of water through a polypropylene yarn cartridge filter. As summarized in *Nieminski et al.,* Applied and Environmental Microbiology, 61(5): 1714-1719, 1995, in the ASTM method, after sampling, particulates from the cartridge filter are extracted by cutting the filter apart and washing the fibers. The extracted particulates are then concentrated by centrifugation. The concentrated particulates are then processed to selectively concentrate parasite cysts and oocysts by floatation in 50-mL tubes on a Percoll-sucrose gradient. Particulates recovered at the interface of the Percoll-sucrose gradient are stained with fluorescently tagged antibodies on 25-mm-diameter, 0.2µm-pore-size cellulose acetate filters. After mounting on slides, the membrane filters are scanned with an epifluorescent microscope for objects having the size, shape and fluorescence characteristic of *Cryptosporidium* oocysts. On finding such objects, the microscope optics are switched to phase contrast to look for internal morphological characteristics inside the detected organisms. Organisms determined to meet the fluorescence detection criteria are counted as presumptive *Cryptosporidium* oocysts. Organisms with the right fluorescence characteristics and shown to have the respective internal morphological characteristics are counted as confirmed *Cryptosporidium* oocysts.

However, the ASTM method is costly and consumes substantial time and labor. It is also not very effective since losses of oocysts occur throughout the procedure. Large numbers of oocysts pass through the filter, or adhere to the filter material and are not recovered. Losses also occur during centrifugation because oocysts are destroyed or resuspended during removal of the supernatant fluid. During a recent blind test of commercial laboratories in the United States, spiked samples were submitted to sixteen laboratories to evaluate their ability to recover and detect *Cryptosporidium* oocysts using the ASTM method. *Six laboratories -- over one third of the total -- failed to recover any parasites, and the remaining ten had an average recovery rate of only 2.8%. Aldom et al.,* Letters in Applied Microbiology 20: 186-187, 1995. A more detailed description of losses of *Cryptosporidium* oocysts during the detection procedure can be found in *LeChevallier et al.,* Applied and Environmental Microbiology 61 (2): 690-697, 1995.

Several studies have been conducted on techniques to improve the efficiencies of methods for concentrating the dilute protozoa. The general protocol in such studies is to spike a water sample with a known amount of *Cryptosporidium* oocysts and determine the percentage of oocysts recovered for each method tested.

A number of studies evaluated other types of filtration, including vortex-flow, cross-flow, and sand column filtration. *Whitmore et al.,* Wat. Sci. Tech. 27 (3-4): 69-76, 1993. In the *Whitmore et al.* study, the vortex-flow filtration technique gave fairly consistent recoveries of 30% to 40%. While this retention rate is superior to that of the ASTM method, the comparatively long process times would prevent the use of this method for monitoring purposes. The crossflow filtration module also gave relatively good recoveries (approximately 40-80%) at moderately high flow rates. The laboratory scale sand columns evaluated gave satisfactory retention within the column material at low flow rates, but were judged inadequate for monitoring purposes because of the poor retention of oocysts within the column matrix at realistic flow rates. Another study tested a filter matrix dissolution method to recover *Cryptosporidium* oocysts from water. The average recovery rate observed was 70.5%. *Aldom et al.,* Letters in Applied Microbiology 20: 186-187, 1995. *Cryptosporidium* oocysts have also been concentrated from water by "sweeping" water with a settling calcium carbonate precipitate. *Vesey et al.,* Journal of Applied Bacteriology 75: 82-86, 1993. The *Vesey et al.* study resulted in a 68% recovery of oocysts from seeded samples of deionized, tap, and river water.

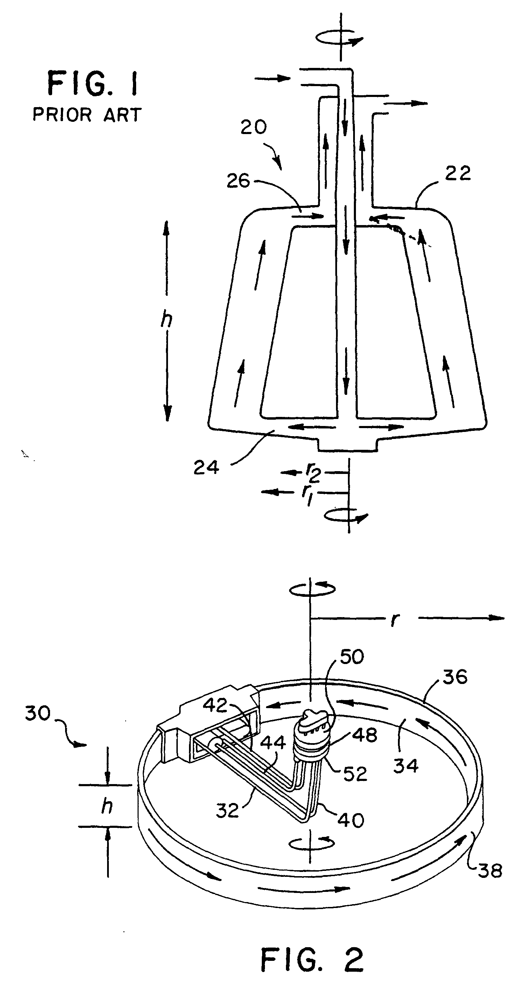

Continuous flow bowl centrifugation has also been studied as a means to concentrate dilute concentrations of *Cryptosporidium* oocysts. An exemplary continuous flow bowl centrifuge commonly referred to as a Latham bowl centrifuge is shown at **20** in **FIG. 1**. Liquid is introduced into the end (top or bottom) of a spinning bowl **22** from an axially-directed input line **24** with length ***r1*** located near the rotational axis of the bowl **22**. As the liquid flows up the height ***h*** of the bowl **22**, centrifugal force forms a density gradient in the liquid with the densest (heaviest) matter (sand or other detritus) located near the wall of the bowl **22** and the lighter matter (e.g., water) being located closer to the rotational axis. As liquid is introduced into the bowl **22**, the lighter portion of the liquid is emptied at the same flow rate from an axially-situated and axially-directed exit line **26** with length ***r2*** which exits the end of the bowl **22** opposite the input line **24**. Continuous flow bowl centrifugation has been used in conjunction with heating to kill bacteria present in beverages, as described by U.S. Patent 3,217,982 to *Wilsmann et al.* However, studies evaluating use of a continuous flow bowl centrifuge for concentrating protozoan parasites have not described increases in recovery rates. The *Whitmore et al.* studies cited above evaluated use of a bowl-type continuous flow centrifuge for concentration of oocysts and reported recovery between 11% to 31.2%. *Goatcher et al.,* American Society of Macrobiology Abstracts Q-212, 1995 also tested a bowl-type continuous flow centrifuge system and reported obtaining recovery rates of between 2 to 20 times those observed with conventional filtration methods; therefore, such recovery rates are still well under 50%.

In view of the importance of protecting water supplies from pathogenic organisms such as protozoan parasites, and the lack of apparata or methods for concentrating and/or detecting such organisms with high recovery rates, it is clearly desirable to develop improved methods and apparata for reliably concentrating waterborne pathogenic organisms.

### Summary of the Invention

The present invention, which is defined by the claims set out at the end of this disclosure, is directed to the concentration of dilute densities of pathogenic organisms, e.g., protozoan parasites such as *Cryptosporidium,* from water. One aspect of the invention concerns a method of concentrating waterborne pathogenic organisms from water potentially contaminated by dilute densities of the organisms wherein the water is fed into an elongated flow path, e.g., a flow path defined by a channel or duct. The flow path includes a separation section wherein the water flow is oriented in directions substantially tangential with respect to a rotational axis. The flow path is then subjected to centrifugal forces by spinning it about the rotational axis in such a manner that the centrifugal forces are oriented substantially perpendicular to the separation section. This highly concentrates pathogenic organisms within the water flowing within the separation section. As will be illustrated below by experimental results, in some cases 100% recovery of *Cryptosporidium* oocysts has been measured.

Examples of apparata for performing this method are channel centrifuges, e.g., the IBM (COBE) model 2997 channel-type blood centrifuge (formerly made by International Business Machines, Armonk, New York, USA and now made by COBE, Lakewood, Colorado, USA) or the COBE Spectra channel-type blood centrifuge. Such channel centrifuges, which are discussed at greater length later in this disclosure, utilize hollow hoop-shaped separation channels with the rotational axis being situated at the center of the hoop. Radially-directed input and exit lines are connected to the separation channel in much the same manner as spokes leading to a wheel. Liquid supplied to the centrifuge travels radially outward in the input line, enters the separation channel and traverses its circumference, and then travels radially inward through the exit line. When the separation channel is spun about the rotational axis, centrifugal forces oriented perpendicular to the channel are generated.

Another aspect of the invention concerns a method of concentrating waterborne pathogenic organisms from water by feeding the water into an elongated flow path which includes a separation section wherein the water flow follows an arcuate path defined about a rotational axis. Pathogenic organisms within the water in the separation section are then concentrated by spinning the flow path about the rotational axis, thereby creating centrifugal forces substantially perpendicular to the separation section. Again, exceptional recovery rates of up to 100% have been achieved. This aspect of the invention may be practiced by utilizing the channel centrifuges noted above, wherein the hoop-like channel serves as the separation section. Alternatively, it may be practiced by use of channels in the shape of ellipses, helixes, parabolic sections, or channels of other shapes which orbit or curve about the rotational axis.

A further aspect of the invention involves a method of concentrating pathogenic organisms from water by feeding the water into a flow path which includes a separation section resting substantially within a plane (or planes) oriented generally perpendicular to the rotational axis. The separation section has a length greater than 2r, where r is the average of the maximum and minimum radial distances of the flow path from the rotational axis. Pathogenic organisms within the water in the separation section are then concentrated with up to 100% recovery rates by spinning the flow path (including the separation section) about the rotational axis, thereby subjecting it to centrifugal force. The aforementioned hoop-like channel centrifuge provides an example of a pre-existing device which may be used to practice this embodiment of the method.

The various aspects of the invention summarized above have less process and apparatus/material costs, consume less time and labor, and are vastly more effective than any previously known methods and apparata for detecting pathogenic organisms such as *Cryptosporidium* in water. Accurate counts of contamination levels are now possible, thereby allowing effective monitoring and treatment plans to be installed. Treated water can be continuously sampled as it is being produced, thereby providing an up-to-date measure of water quality as compared to the delayed warning produced by the current method of spot-checking water supplies. Since the invention allows water treatment plants to regard organism counts with a high level of confidence, they will no longer be forced to undergo expensive repeated testing and/or remediation measures as a precautionary measure when questionably low counts are measured. Additionally, in view of the fact that the current test standard is highly unreliable (as noted by *Aldom et al., supra*), the invention can prevent the hardship endured by communities when outbreaks occur. Other advantages and features of the invention will be apparent from the remainder of this disclosure.

### Brief Description of the Drawings

**FIG. 1** is a schematic cross-sectional view of a prior art continuous flow bowl centrifuge.

**FIG. 2** is a perspective view of the channel assembly of a continuous flow channel centrifuge.

**FIG. 3** is a schematic diagram of a section of the channel of **FIG. 2** illustrating the channel input and outlet lines in greater detail.

**FIG. 4** is a perspective view of another embodiment of a channel assembly encompassed by the invention.

**FIG. 5** is a schematic diagram of a section of the channel of **FIG. 4** illustrating the channel input and outlet lines in greater detail.

**FIG. 6** is a schematic diagram of a section of an alternate embodiment of a channel illustrating the channel input and outlet lines in greater detail.

**FIGS. 7-11** are perspective views of alternate embodiments of channel assemblies encompassed by the invention.

**FIG. 12** is a schematic diagram of a section of an alternate embodiment of a channel illustrating the channel input and outlet lines in greater detail.

### Detailed Description of the Invention

### Continuous Flow Channel-Type Centrifuges

A separation channel assembly from a typical channel-type continuous flow centrifuge, used to concentrate waterborne pathogenic organisms according to the present invention, is depicted in **FIG. 2** at reference numeral **30**. The channel assembly **30** depicted in **FIG. 2** is part of an IBM model 2997 blood separation channel centrifuge. The division of IBM which manufactured the channel assembly **30** pictured in **FIG. 2** is now owned by COBE Corporation, Lakewood, Colorado, USA. References of interest regarding the IBM model 2997 channel centrifuge (and/or centrifuges like it) are U.S. Patents 4,010,894, 4,094,461, 4,387,848 and 4,430,072 to *Kellogg et al.;* U.S. Patents 4,386,730, 4,439,178, 4,447,221 and 4,708,712 to *Mulzet;* U.S. Patent 4,647,279 to *Mulzet et al.;* U.S. Patent 4,900,298 to *Langley;* U.S. Patent 5,496, 265 to *Langley et al.;* U.S. Patent 4,850,995 to *Tie et al.;* U.S. Patent 4,419,089 to *Kolobow et al.;* and the references cited within these patents.

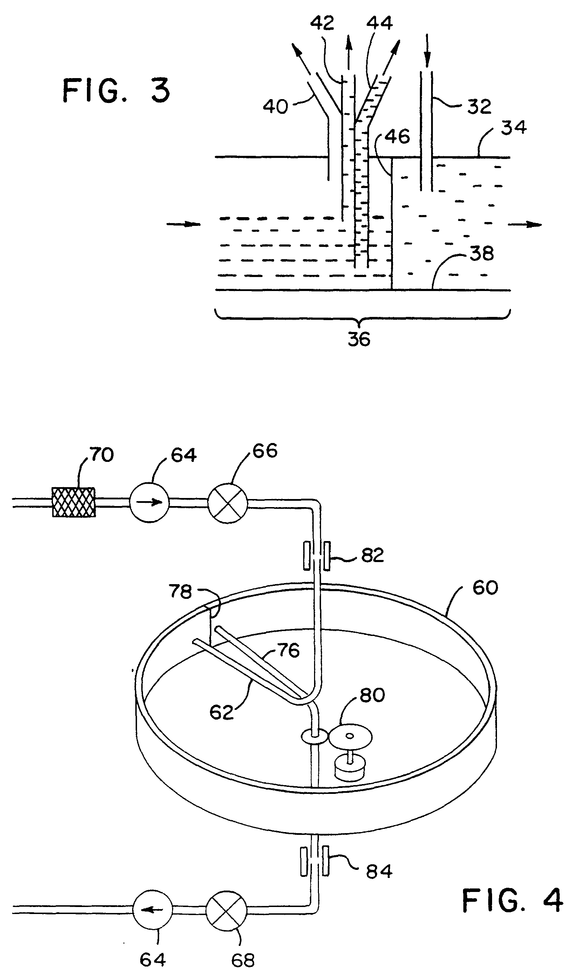

When the channel assembly **30** of **FIG. 2** is used as blood separator, blood from a donor's arm is continuously pumped from a radially directed input line **32**, through the inner channel wall **34**, and into hollow channel **36** as the channel assembly **30** rotates. The various blood components (e.g., plasma and red blood cells) are flung toward the outer channel wall **38** by centrifugal force, and they therefore separate according to their specific gravity as the blood moves around channel **36** from the input line **32** to several exit lines **40**, **42**, and **44** (shown in greater detail in **FIG. 3**). The blood cannot flow directly from the input line **32** to the output lines **40/42/44** and is forced to traverse the entirety of the channel **36** owing to the barrier **46** situated between the inlet and outlet lines **32** and **40/42/44.** To collect the various layers of blood, the exit lines **40/42/44** are positioned at different radial locations spaced from the inner wall **34** of channel **36**. Plasma, leukocytes (white cells), and red blood cells are each collected through respective exit lines **40**, **42**, and **44**.

The structure and operation of continuous flow channel centrifuges may be better understood if they are compared and contrasted to the structure and operation of continuous flow bowl centrifuges. In the channel **36** of **FIG. 2**, blood flow is in an arcuate path centered about the rotational axis. Flow occurs entirely in planes perpendicular to the rotational axis, and the total length of the flow path subjected to centrifugal force of any appreciable magnitude is approximately ***2r*** (in the input and exit lines **32** and **40/42/44**) plus ***2π******r*** (in the channel **36**), wherein ***r*** is the radial distance between the rotational axis and the inner wall **34** of the channel **36**. The centrifugal force is exerted perpendicular to the channel **36**, and most of the separation between heavy and light blood fractions occurs along its length, wherein the direction of blood flow is tangential to the channel **36** and in a direction perpendicular to the rotational axis.

In comparison, a continuous flow bowl-type centrifuge such as the one shown in **FIG. 1** does not use a flow path curving about the rotational axis; rather, the flow path of any element of blood entering the centrifuge will be generally along a plane coincident with the rotational axis. The overall flow path has a length of ***2r*** plus ***h****,* the ***2r*** flow path being in the input and exit lines **24** and **26** and oriented in planes perpendicular to the rotational axis, and the h flow path being directed along the height of the bowl **22** and oriented in planes parallel to the rotational axis. Most of the separation between heavy and light blood fractions occurs along the walls of the bowl **22**, wherein the direction of blood flow is generally parallel to the rotational axis.

A unique design feature of certain channel assemblies (such as the model 2997) is their rotating seal assembly **48**. It consists of a stationary top half **50** and a bottom half **52** which rotates with the channel **36**. The surfaces where the two halves **50** and **52** meet have four concentric matching grooves (not shown) that correspond to whole blood input line **32** and the three exit lines **40**, **42**, and **44**. Whole blood and blood components are transferred from stationary to moving parts through the four grooves. The hydrodynamic design of the grooves and the viscosity of blood prevent the liquid from leaking between the junction of the two halves **50** and **52** of seal assembly **48**, which is open to the atmosphere. Because bloodborne pathogens could potentially leak into or from the seal, blood cell separators using a rotating seal assembly have become obsolete.

### Use of the Separation Channel to Concentrate Pathogenic Organisms

To concentrate pathogenic organisms such as *Cryptosporidium* and other protozoan parasites from potentially contaminated water in accordance with the invention, water may be fed into the channel assembly **30** illustrated in **FIGS. 2** and **3**, which is operated as a simple continuous-flow centrifuge. However, prior to sending water through the channel, several preliminary steps are preferred.

First, the channel **36** and associated tubing are preferably primed with water containing a surfactant, for example 0.001 % Tween 80 or Tween 20 (Sigma Chemical Co., St. Louis, Missouri, USA), to enhance removal of the collected material containing the organisms after centrifugation is completed. Priming may be conducted with an output pump (not shown), the plasma pump built into the centrifuge, or a separate additional peristaltic pump (as discussed below). Priming with a surfactant is not performed if the channel assembly **30** is used for blood separation.

Second, when a centrifuge using the channel assembly **30** is used as a blood separator, donor safety sensors (not shown) within the centrifuge test the tubing of the channel assembly **30** for air emboli and pressure irregularities. When the channel assembly **30** is instead used to concentrate pathogenic organisms, the sensors and unnecessary pumps and circuits may be disabled or removed.

Third, when channel assemblies **30** having the split-seal arrangement **48** are used, water containing 50% glycerin (or another viscosity-modifying substance) is pumped into the saline line (not shown) of rotating seal assembly **48** using a lubricant pump (not shown) operating at a speed of about 2.5 rpm. The glycerin creates a viscous barrier between water and air at the junction of the two halves **50** and **52** of seal assembly **48** to prevent leaking. Glycerin is not used when split-seal channel centrifuge assemblies are used as blood separators because blood is normally sufficiently viscous to move between stationary and moving halves **50** and **52** without leaking. If channel assemblies with newer valves (i.e., non-split-seal valves) are used, the viscosity modification is unnecessary.

Water potentially contaminated by dilute densities of pathogenic organisms is then fed into channel **36** via whole blood input line **32** at a flow rate in the range of between about 70 mL/min to 500 mL/min, and preferably about 150 mL/min. The water is centrifuged at a speed such that the relative gravitational force inside the channel **36** is about 900xg, which equates to approximately 2400 rpm for the IBM model 2997 channel assembly **30**. Centrifugation is performed for a period of time sufficient to process a predetermined sample volume.

After centrifugation, the organisms, inorganic sediment, and detritus are retained in channel **36** rather than being pumped out, as is customary with red blood cells when blood separation is performed. The supernatant (water) is pumped out via plasma exit line **40**.

### Experimental Results

Initial experiments to test the feasibility of channel assembly **30** for concentrating and collecting pathogenic organsisms from water will now be summarized. An aqueous medium was spiked with a microorganism, usually *Cryptosporidium,* at a known concentration. The medium was then centrifuged by use of a continuous flow channel centrifuge. Except for experiments 6, 7 and 8 summarized below, the aforementioned model 2997 channel assembly **30** was used.

Several tests were conducted at the maximum sample feed rate of the built-in plasma pump, about 70 mL/min. Other tests used a separate peristaltic pump which was not part of channel assembly **30** and which was capable of providing a maximum sample flow rate of 500 mL/min. Centrifugation took a few minutes to several hours depending on the water sample volume. The processing time is normally calculated as the sample volume divided by the sample feed rate, and in general, the centrifugation time necessary to concentrate protozoa from typical water samples will be on the order of about 1/2 to 4 hours when the model 2997 channel assembly **30** is used. For smaller samples, lesser times are sufficient.

After centrifugation was complete, the input and exit lines **32** and **40/42/44** from seal assembly **48** to channel **36** were carefully clamped, the channel **36** removed from the rotor, and the contents drained into a beaker. The channel **36** was carefully cut in half so that its contents were not spilled, and its cut ends were then clamped with VISE GRIP pliers. Each half of the channel **36** was then filled with 0.01% Tween 80 and its ends were clamped shut. The channel **36** was then shaken vigorously and placed on a laboratory vortex to dislodge any *Cryptosporidium* that may have adhered to the channel. This rinsing procedure was conducted several times. The concentrate and all rinses were combined. For these feasibility studies, no further steps were taken to separate *Cryptosporidium* from debris in the water. The combined concentrate and rinses were then examined for *Cryptosporidium* oocysts and/or other cysts by use of the Merifluour Detection Kit, an immunofluorescent assay produced by Meridian Diagnostics, Cincinnati, Ohio. All results are reported as percent recovery, i.e. total number of organisms recovered in the channel divided by the number added to the medium, multiplied by 100.

### EXPERIMENT 1: Recovery of Cryptosporidium Oocysts from Phosphate-Buffered Saline Solution

Phosphate-buffered saline (PBS) is a simpler aqueous medium than naturally occurring surface or ground water, and therefore would presumably present optimum conditions for concentrating *Cryptosporidium* oocysts by a continuous flow channel centrifuge. Apart from testing oocyst recovery at these theoretically optimum conditions, the experiment tested whether recovery efficiency varied with oocyst concentration, an important consideration given the range of oocyst densities that exist in the environment.

*Cryptosporidium* oocysts from the positive control of the Merifluour Detection Kit were diluted with 10% formalin to make a working stock solution having a concentration of approximately 5000 oocysts/mL. Oocysts were spiked into 2 or 5 L of PBS to achieve the following four target concentrations: 1000, 100, 20 or 5 oocysts/L. The IBM 2997 blood cell separator was used as the continuous flow channel centrifuge. The centrifuge feed rate was set at 70 ml/min, and the rotor speed was 2200 rpm (relative centrifugal force of 740xg). The total centrifuge time was 1/2 to 1 hour per sample. The recovery efficiency of the centrifuge was tested 3 to 5 times at each oocyst concentration.

The material from the channel was tested for oocyst presence. The supernatant remaining in the channel after centrifugation was also collected, concentrated by bulk centrifugation at 10000xg for 15 min, and the pellets were analyzed for oocysts in the same manner. The results show that *Cryptosporidium* recoveries of greater than 69% were achieved, and that, most importantly, a recovery of nearly 100% was achieved for the most dilute oocyst concentration (5 oocysts/L):

| **Oocysts/Liter** | **No. of Tests** | **Mean % Recovery** | **Std. Dev. % Recovery** |
|---|---|---|---|
| 100 | 3 | 84.4 | 15.7 |
| 1000 | 4 | 68.5 | 5.7 |
| 20 | 5 | 93.8 | 33.0 |
| 5 | 3 | 98.8 | 16.7 |

### EXPERIMENT 2: Recovery of Cryptosporidium Oocysts from Pond Water

To more closely simulate the conditions under which the invention would operate when concentrating naturally occurring *Cryptosporidium,* live oocysts were spiked into pond water. Recovery efficiencies were compared with those attained using PBS and between two oocyst concentrations in pond water.

Purified live *Cryptosporidium* oocysts were obtained from Parasitology Research Labs (Phoenix, Arizona, USA) and diluted with distilled water for a working stock concentration of approximately 15,000 oocysts/mL. Oocysts were spiked by adding the appropriate volume of stock solution to 100 mL of pond water, fixing the *Cryptosporidium* with formalin (10% final concentration), then mixing the 100 mL aliquot in the larger pond water volume to be centrifuged. These steps were taken to insure that the organisms were killed while minimizing the amount of added formalin.

*Cryptosporidium* oocysts were spiked into 2, 10, or 15 liters of pond water, collected from the same local pond (Pond 1) on the day of centrifugation. Water was collected from the pond surface with 5 gallon buckets where the water depth was at least 50 cm. Collection of bottom sediments was avoided. The sampled pond water had 49 mg/L total solids and turbidity of 2.1 NTU.

Ten liters of water from Pond 1, without added oocysts, were centrifuged as a control. Indigenous *Cryptosporidium* oocysts were not found. Oocyst target concentrations were 1000 or 100 oocysts/L. The centrifuge feed rate was set at 70 mL/min, and the rotor speed was 2400 rpm (generating approximately 900xg of force). Centrifuge times were 1/2 to 3 1/2 hours. The recovery efficiency was tested 3 times at each oocyst concentration.

The results, presented below, show recoveries of 100% for the most dilute oocyst concentration (100 oocysts/L). The percentage recoveries were calculated based on the concentration of oocysts retained in the channel as compared to the amount of the initial spiking. The supernatants were also examined for oocysts not retained by the centrifuge. Except for the sample feed rates of 250 and 500 ml/min described in Example 3 below, no oocysts were detected in any of the supernatants. Thus, it is most likely that organism separation in the channel was close to 100%, and the apparent loss of organisms from the concentrate to be tested probably arises due to other steps in the process (e.g., during use of the immunofluorescent assay).

| **Oocysts/Liter** | **No. of Tests** | **Mean % Recovery** | **Std. Dev. % Recovery** |
|---|---|---|---|
| 100 | 3 | 102.9 | 5.6 |
| 1000 | 3 | 90.3 | 10.8 |

### EXPERIMENT 3: Recovery of Cryptosporidium Oocysts as a Function of Sample Feed Rate

The time required to concentrate oocysts by continuous flow centrifugation depends on the sample volume and the sample feed rate into the centrifuge. Given the maximum relative centrifugal force generated by the blood cell separator (900xg), this experiment was conducted to determine the maximum sample feed rate that could be used without reducing recovery efficiency.

Purified live *Cryptosporidium* oocysts were used as described in Experiment 2. *Cryptosporidium* oocysts were spiked into 2 or 10 L of water from Pond 1. The target concentration of oocysts was 1000/L for all centrifugation runs. Percent recovery was measured at three sample feed rates: 150, 250, and 500 ml/min. Centrifugation times were 4 minutes to 1 hour.

Samples were pulled into the channel at the desired rate by a peristaltic pump (Cole Parmer model 7553-20) placed downstream from the channel. The built-in centrifuge pumps were bypassed because their maximum pumping rate is only 70 ml/min. Recovery efficiencies were measured 3, 4 or 5 times at each sample feed rate.

The results, presented below, show recovery rates of between *45-95* %, with the highest recovery achieved at the lowest flow rate tested (150 mL/min).

| **Pump Speed mL/min** | **No. of Tests** | **Mean % Recovery** | **Std. Dev. % Recovery** |
|---|---|---|---|
| 150 | 3 | 94.4 | 5.9 |
| 250 | 3 | 70.3 | 6.0 |
| 500 | 5 | 43.3 | 5.5 |

### EXPERIMENT 4: Recovery of Cryptosporidium From Pond Water, Sample Feed Rate = 150 mL/min

Having observed in Experiment 3 that oocysts could be recovered efficiently when the sample feed rate is 150 mL/min, this example tested whether the recovery efficiency changed with oocyst concentration at that feed rate.

Purified live *Cryptosporidium* oocysts were used as described in Experiment 2. *Cryptosporidium* oocysts were spiked into 10 or 25 L of water from another local pond, here denoted Pond 2. For Pond 2, total solids were 182 mg/L, and turbidity was 8.3 NTU.

Ten liters of water from Pond 2 without added oocysts was centrifuged as a control. Indigenous *Cryptosporidium* oocysts were not found.

Target concentrations were 1000, 100 or 20 oocysts/L. The independent peristaltic pump pulled the samples into the IBM 2997 centrifuge at a flow rate of 150 mL/min. The rotor speed was 2400 rpm (900g). The time for centrifugation was one to three hours. Recovery efficiencies were measured 3 or 4 times at each *Cryptosporidium* concentration.

Supernatants were tested as described in Experiment 1. The results, presented below, show *Cryptosporidium* recoveries of between 80% and nearly 100%, with the highest recovery achieved at an oocyst concentration of 100 oocysts/L.

| **Oocysts/Liter** | **No. of Tests** | **Mean % Recovery** | **Std. Dev. % Recovery** |
|---|---|---|---|
| 100 | 4 | 100.8 | 15.1 |
| 1000 | 3 | 97.2 | 9.6 |
| 20 | 3 | 77.9 | 17.9 |

### EXPERIMENT 5: Recovery of Giardia Cysts From Pond Water

The centrifugation process was tested with *Giardia lamblia,* another waterborne protozoan pathogen, to assess whether the centrifuge could efficiently concentrate pathogens larger than *Cryptosporidium.*

Purified live *Giardia* cysts were obtained from Parasitology Research Labs (Phoenix, Arizona, USA) and diluted with distilled water to approximately 22,000 cysts/mL. Cysts were added to water from Pond 2 following the same procedure for *Cryptosporidium* described in Experiment 2.

Ten liters of water from Pond 2, without added cysts, were centrifuged as a control. Indigenous *Giardia* cysts were not found. *Giardia* cysts were spiked into 10 or 30 L of pond water. Target concentrations of *Giardia* cysts were 1000, 100 or 20/L. The centrifuge setup was the same as described in Experiment 4. Sample feed rate was 150 mL/min, and the rotor speed was 2400 rpm. Centrifuge time was 1 to 3 1/2 hours. Recovery efficiencies were measured 3 times at each *Giardia* concentration.

The results, presented below, show recoveries of *Giardia* cysts to be between 90-100%.

| **Target Concentration Oocysts/Liter** | **No. of Tests** | **Mean % Recovery** | **Std. Dev. % Recovery** |
|---|---|---|---|
| 100 | 3 | 94.6 | 5.2 |
| 1000 | 3 | 104.2 | 5.7 |
| 20 | 3 | 97.0 | 10.5 |

### EXPERIMENT 6: COBE Spectra Centrifuge

The COBE Spectra centrifuge is a newer continuous flow channel centrifuge for use in blood cell separation. Unlike the IBM 2997 centrifuge discussed above, there is no rotating ceramic seal. The channel itself has a hoop-shaped geometry similar to the channel **36** of **FIG. 2**.

Purified live *Cryptosporidium* oocysts were used as described in Experiment 2. Oocysts were spiked into 5 or 7.5 L of water from Pond 2. The oocyst target concentration was 100/L for all centrifuge runs. The input pump of the COBE Spectra centrifuge was set at 150 mL/min, and the rotor speed was 2400 rpm (900xg). The recovery efficiency was tested 3 times.

Using the COBE centrifuge to concentrate *Cryptosporidium,* 100% of the spiked *Cryptosporidium* oocysts were recovered.

| **Oocysts/Liter** | **No. of Tests** | **Mean % Recovery** | **Std. Dev. % Recovery** |
|---|---|---|---|
| 100 | 3 | 103.3 | 14.8 |

### EXPERIMENT 7: Recovery of Cryptosporidium Oocysts as a Function of Water Turbidity

An effective method for concentrating pathogenic organisms from water must work over a wide range in water turbidity. In this experiment turbidity was manipulated by concentrating seston in pond water by bulk centrifugation (10,000xg for 15 minutes). For example, to increase turbidity 10-fold above ambient, 10 volumes of pond water were concentrated and the seston pellets added and mixed to the unit volume to be spiked with oocysts. Turbidity was measured using a fluorescence spectrophotometer (Hitachi Model F-4500, excitation and emission wavelengths = 500.0 nm, slit width = 2.5 nm, and PMT voltage = 400 V). Oocysts were spiked into 7.5 L pond water, as described in Experiment 2, at a target concentration of 100 oocysts/L. Oocysts were recovered with the COBE Spectra centrifuge (rotor speed = 2400 rpm or approximately 900xg, sample feed rate = 150 mL/min).

The results, presented below, show that percent recovery of oocysts did not vary with turbidity levels between 2.6 and 30.8 NTU (F statistic, p>0.05, comparison based on count aliquots (n=6) because turbidity levels could not be replicated).

| **Turbidity (NTU)** | **No. of Tests** | **Mean % Recovery** | **Std. Dev. % Recovery** |
|---|---|---|---|
| 2.6 | 6 | 84.9 | 17.0 |
| 4.8 | 6 | 81.5 | 27.7 |
| 8.6 | 6 | 92.2 | 20.1 |
| 15.7 | 6 | 93.8 | 29.9 |
| 20.1 | 6 | 101.2 | 40.9 |
| 30.8 | 6 | 82.3 | 29.3 |

### EXPERIMENT 8: Recovery of Cryptosporidium from Large Pond Water Volumes

Given that waterborne pathogenic organisms may occur in dilute densities, an effective concentration method must be able to process a large sample volume, on the order of 100 liters. In this experiment live and purified *Cryptosporidium* oocysts were spiked into 100 L of pond water. Spiking was conducted as described for Experiment 2. The target density was 100 oocysts/L. Oocysts were recovered with the COBE Spectra centrifuge (rotor speed = 240 rpm or approximately 900g, sample feed rate = 150 mL/min). Centrifugation time for this rotor speed and sample feed rate was 11 hours.

Mean percent recovery of *Cryptosporidium* oocysts from 100 L of pond water was 94.7 ± 9.7% (mean ± 1 standard deviation, n=3). The recovery efficiency was not significantly different than recovery with the centrifuge and a sample volume of 5 to 7 L (two-tailed t test, p > 0.05), as performed in Experiment 6.

### Modifications and Alternate Embodiments

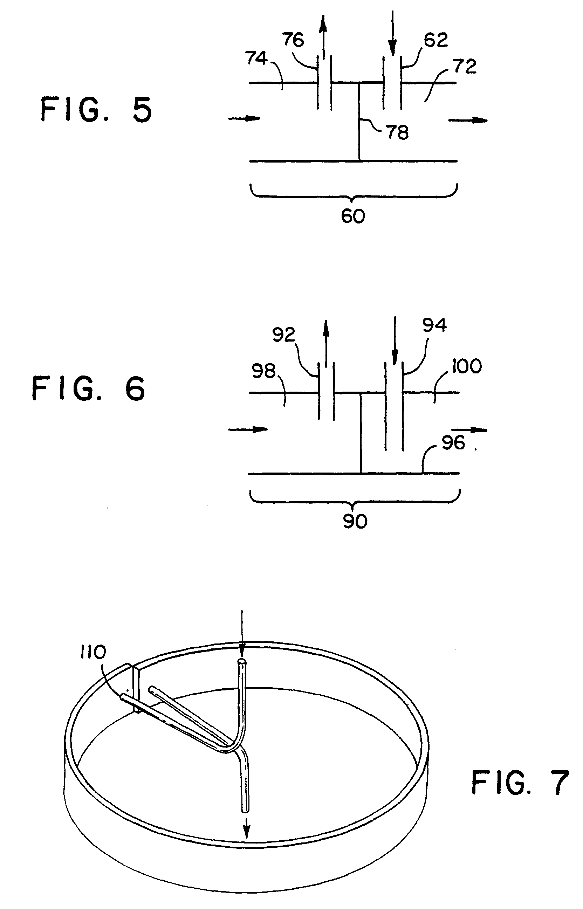

Another embodiment of a channel suitable for use in concentrating dilute densities of pathogenic organisms from water is illustrated in **FIGS. 4** and **5**. The channel **60** includes an input line **62** downstream of a pumping means **64** for supplying potentially contaminated water to the channel **60**. This pumping means **64** is a source of static or dynamic pressure head, i.e., a pump, tank, or other water sources which generate water flow into the input line **62**. Pressure restriction/flow control valves can be provided upstream and/or downstream of the channel **60**, as illustrated at **66** and **68**. A coarse sediment filter **70** may also be placed upstream from the channel **60**, to remove any gross impurities from the input line **62** before they enter the channel **60** and clog it. Water flows from the input line **62**, into the channel entry **72**, and then through the channel **60** (not fully shown in FIG. 5) to the channel end **74**. The water then exits the channel **60** from the exit line **76**, which may have a downstream pumping means in addition to (or in place of) the pumping means **64** upstream of the input line **62**. As shown in **FIG. 5**, the channel end **74** and channel entry **72** are separated by a barrier **78** so that water can only reach the exit line **76** from the input line **62** after flowing through the entirety of the channel **60**. The centrifuge drive is depicted at **80**. The principal difference between the apparatus of **FIGS. 4** and 5 from that of **FIGS. 2** and **3** is that the channel **60** need only include a single exit line **76** rather than several, since separation of the input water into multiple fractions of different density will generally not be of interest. Additionally, the split-seal valving arrangement (and thus the necessity for adding a viscosity modifier) is avoided by having the input and exit lines **62** and **76** extend axially to join high-speed rotary seals **82** and **84**.

Another embodiment of a channel suitable for use in concentrating dilute densities of pathogenic organisms from water is illustrated in **FIG. 6**. The channel **90** of **FIG. 6** differs from that of **FIGS. 2-3** in several important respects. First, it need only include a single exit line **92** rather than several. Second, the input line **94** is located radially outward of the exit line **92**, rather than at a radially inward or radially equivalent position. The use of a radially inwardly located exit line **92** helps to prevent organisms from being carried directly to the input line **94** to the exit line **92** by the water flow, or from being diverted to the exit line **92** due to turbulence in the channel, because the organisms will be more likely to settle radially outwardly to the outer channel wall **96** by the time they reach the channel end **98**. In other words, immediately upon entering the channel entry **100** via the input line **94**, the heavier organisms will begin traveling toward the outer channel wall **96** and are less likely to defeat the force gradient to exit the exit line **92**. The input line **94** can be radially positioned inward of the exit line **92** by simply fixing the input and exit lines **94** and **92** in this location, as shown in **FIG. 6**. Alternatively (or additionally), this be done by modifying the shape of the channel so that the channel end **98** is located radially inward of the channel entry **100**, as by modifying the channel into a generally spiral shape rather than an annular shape. In this configuration, which is shown in **FIG. 7**, the highest centrifugal force vectors are exerted on the organisms near the channel entry **110**.

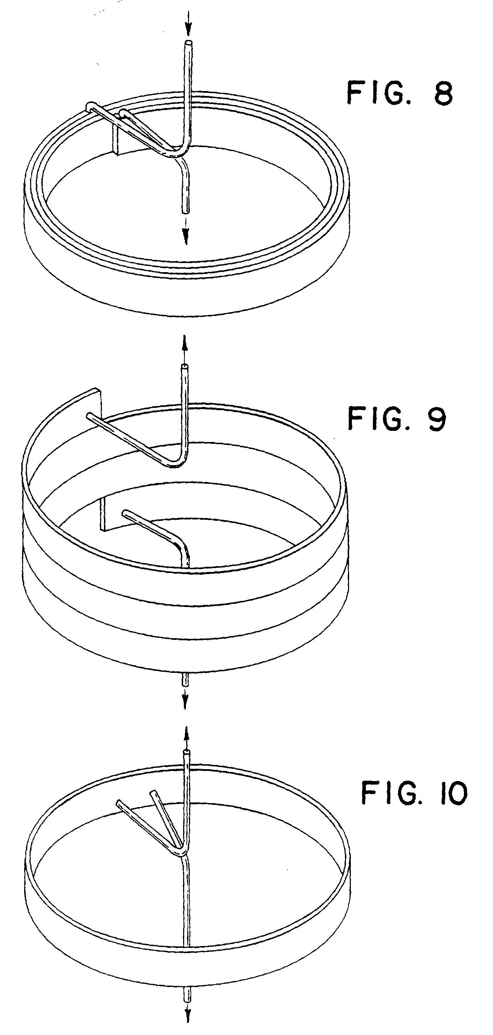

Regarding the modification of the channel into a spiral shape, it is noted that embodiments such as that of **FIG. 8** are possible wherein a horizontal spiraling configuration allows for a channel of much greater length, and thus much greater residence time for the water flowing therein. The vertical spiraling configuration of **FIG. 9** is another possibility. The longer channels provided by these embodiments may help in further concentration of organisms.

It is also possible to modify the shape of the channel into a generally elliptical configuration, as shown in **FIG. 10**. Configurations of this type may be helpful in that higher concentrations of organisms may gather at the areas wherein the channel intersects the major axes of the ellipse, since these are the areas where the maximum centrifugal forces are exerted on the water.

In similar fashion, it may be helpful to form a series of depressions in the outer wall of the channel, as exemplified in the channel **120** of **FIG. 11**. These depressions form traps **122** which may catch and retain organisms to a greater degree than the surrounding regions on the outer channel wall **124**, which are located more radially inward.

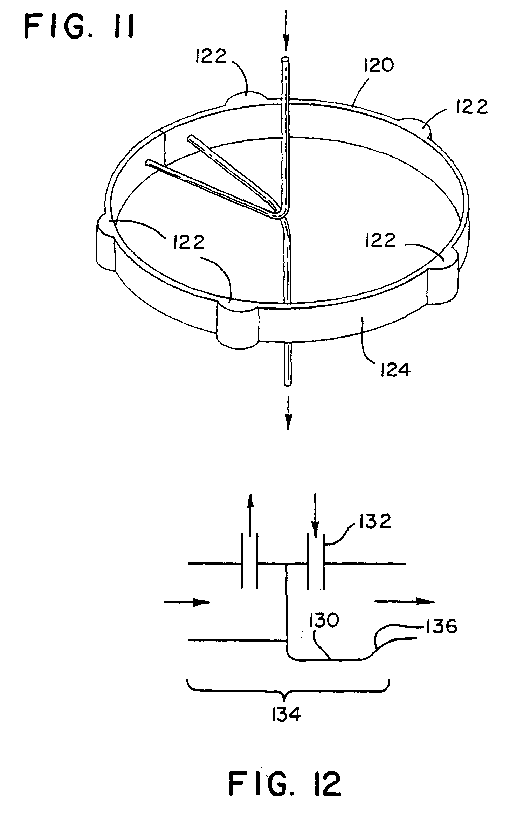

Strategic placement of these traps may lead to other beneficial results as well. As an example, **FIG. 12** provides a closer view of a trap **130** opposite the input line **132**. A trap **130** at or near this location is recommended when more turbid water is being tested, not so much for the purpose of capturing organisms, but for capture of sand and other very heavy detritus. Upon entering the channel **134**, these heavy gross impurities will rapidly fall into the trap **130**, thus providing for "cleaner" collection of organisms in downstream channel areas. However, if any traps **130** or other discontinuities are formed in any of the walls of the channel **134**, care must be taken to form them in such a manner that they do not generate undue turbulence in the water flow (hence the gently sloping downstream surface **136** of the trap **130** shown in **FIG. 12**). Such turbulence may dislodge organisms from the channel walls or redivert a settling organism into a flow path located further radially inward, and this could result in organisms escaping the channel **134**.

It may also be helpful to form the aforementioned traps **130/136** with transparent walls so that the contents of the traps **130/136** will be visible to centrifuge operators when the channel is at rest. Such traps would also be visible when the channel is spinning if used in conjunction with a strobe.

It is noted that while the invention has generally been described with reference to a channel having a duct-like fully enclosed flow passageway, the channels described in this disclosure are not confined to this form. Consider, for example, that the inner channel wall **34** of the channel **36** of **FIGS. 2** and **3** is not absolutely necessary if fluid addition, fluid withdrawal, and channel flushing all occur while the channel **36** is rotating at high speed: since the liquid is flung radially outward against the outer channel wall **38** by centrifugal force, the inner channel wall **34** would not restrain the liquid or have any other practical effect on the liquid. Similarly, the overall shape of the channel -- whether circular, elliptical, or spiral, and whether oriented entirely within the same plane or in multiple planes (as in the vertical spiral configuration illustrated in **FIG. 9**) -- is not critical, though it can provide desirable results if properly chosen. What is regarded to be important is the introduction of potentially-contaminated water into a continuous flow centrifuge wherein the flow is generally tangentially oriented over at least a section of the flow path. (In this disclosure, when a section of a flow path is stated to have "tangential" or "tangentially oriented" flow, this is intended to mean that when this section of the flow path is viewed from a direction coincident with the rotational axis, the instantaneous direction of liquid flow at each location along the section is at least substantially coincident with or parallel to the instantaneous direction of motion of the liquid at that location owing to rotation. As an example, the hoop-like channel of **FIG. 2** has tangential flow.) In a continuous flow centrifuge utilizing generally tangential flow, this is best met by situating the input and exit lines substantially near the ends of the flow path. Thus, the optimal hoop-shaped channel for use in concentrating pathogenic organisms would use closely-spaced input and exit lines connected to the channel, with the channel having a barrier between the input and exit lines to force the water to flow through the full extent of the channel. Such a barrier need not fully close the channel; rather, it need only extend radially inward from the outer channel wall to such an extent that the water, when input at a given flow rate and when subjected to centrifugal forces, resides at a height lower than the height of the barrier (this "height" being measured in the radial direction).

However, there is a factor that is regarded to be of potential importance regarding the form of the channel. It is believed that forming the channel to better maintain laminar flow conditions therein, as by removing any sharp convergences, divergences, or discontinuities within the channel, will result in better recovery of organisms. Turbulence may allow the organisms to defeat centrifugal force gradients within the channel and allow the organisms to escape the channel's exit line. Laminar flow may also be encouraged by maintaining a lower flow rate through the channel, though higher flow rates are of course preferred for obtaining lesser testing times with larger sample throughput.

As noted above, it is believed that longer channels (flow paths) are believed to yield better organism recovery. However, it is conceivable to have a channel with a shorter flow path than the channels described above, as in the case of a channel which occupies only a subsection (arc) of a path curving about the rotational axis, or a circular channel wherein the inlet and exit lines are spaced apart with no barrier situated between so that two shorter flow paths are effectively formed, each having an opposite flow direction. While these would likely result in at least partial recovery of organisms, it is believed that they would not attain the same degree of recovery as a standard hoop-like channel operating under similar conditions. It is suggested in any case that channels used in accordance with the invention have flow paths with lengths greater than ***2r***, where ***r*** is the radial distance from the rotational axis to the inner wall of the channel, and preferably a length approaching ***2πr****.* In the case of spiral, elliptical, or other noncircular channels, this relationship is interpreted to have ***r*** be the average of the maximum and minimum radial distances from the rotational axis to the inner wall of the channel.

At some point, it may be desirable to concentrate pathogenic organisms which are *lighter* than water, as where the organisms have accumulated large amounts of intracellular lipids. In this case, the teachings set out above may still be applied with thought being given to the result to be achieved; for example, the input/exit line arrangement of **FIG. 6** may be modified to have the input line located radially inward of the exit line, the traps of **FIGS. 11** and **12** may be located on the inner channel wall rather than the outer channel wall, and so on.

The invention can be installed in preexisting water treatment facilities to provide sampling of water for pathogenic organisms. For example, the invention can be used in series or parallel with the water intake of a treatment facility to provide continuous sampling for pathogenic organisms, thereby replacing the current time-consuming method of periodically spot-checking the water. In this arrangement, it may be useful to install a known flow restrictor/pressure limiting valve upstream or downstream of the centrifuge to maintain the flow rate at a desired cutoff value, and/or install an upstream coarse sediment filter to avoid potential clogging by gross impurities (as illustrated in **FIG. 4**).

One of ordinary skill in the art will comprehend that while this disclosure has mainly described the application of the invention to concentration of *Cryptosporidium* (and to a lesser extent *Giardia),* the invention is expected to be equally applicable to other concentration of other protozoa, as well as other waterborne pathogenic organisms in general, since generally the same considerations will apply.

It is understood that preferred embodiments of the invention have been described above in order to illustrate how to make and use the invention. The invention is not intended to be limited to these embodiments, but rather is intended to be limited only by the claims set out below. Thus, the invention encompasses all alternate embodiments that fall literally or equivalently within the scope of these claims. It is understood that in the claims, means plus function clauses are intended to encompass the structures described above as performing their recited function, and also both structural equivalents and equivalent structures. As an example, though a nail and a screw may not be structural equivalents insofar as a nail employs a cylindrical surface to secure parts together whereas a screw employs a helical surface, in the context of fastening parts, a nail and a screw are equivalent structures.

## Claims

1. A method of concentrating pathogenic organisms from water comprising the steps of:
a. continuously feeding the water into a flow path, the flow path including a separation section substantially oriented within planes generally perpendicular to the axis of rotation, and wherein the separation section has a length greater than 2r, where r is the average of the maximum and minimum radial distances of the flow path from the rotational axis; and
b. subjecting the flow path to centrifugal forces by spinning the flow path about the rotational axis.

2. A method of concentrating pathogenic organisms from water comprising the steps of:
a. continuously feeding the water into an elongated flow path, the flow path including a separation section wherein the water flow is oriented in directions substantially tangential with respect to a rotational axis; and
b. subjecting the flow path to centrifugal forces by spinning the flow path about the rotational axis, wherein the centrifugal force is substantially perpendicular to the separation section.

3. A method of concentrating pathogenic organisms from water comprising the steps of:
a. continuously feeding the water into an elongated flow path, the flow path including a separation section wherein the water flows in an arcuate path about a rotational axis; and
b. subjecting the flow path to centrifugal forces by spinning the flow path about the rotational axis, wherein the centrifugal force is substantially perpendicular to the separation section.

4. The method of any preceding claim wherein after spinning the flow path, the water therein is tested for the presence of pathogenic organisms.

5. The method of any preceding claim wherein the water flow is laminar throughout substantially all of the separation section.

6. The method of any preceding claim wherein the surfaces defining the flow path are coated with a surfactant.

7. The method of any preceding claim wherein the centrifugal force creates a relative gravitational force within the separation section of at least 900xg.

8. The method of any preceding claim wherein the separation section includes the point on the flow path which is spaced at the maximum radial extent from the rotational axis.

9. The method of any preceding claim wherein the flow path is defined by a channel centrifuge.

10. The method of any preceding claim wherein the pathogenic organisms are protozoa.

11. The method of any preceding claim wherein the flow path includes a water input line and a water exit line, each situated near an end of the flow path.

12. The method of claim 11 wherein the input and exit lines are adjacent each other on the flow path with a barrier resting therebetween.

13. The method of claim 11 wherein at least one of the input and exit lines is substantially radially oriented toward the rotational axis.

14. The method of any of claims 2-13 wherein the separation section has a length greater than 2r, where r is the average of the maximum and minimum radial distances of the flow path from the rotational axis.

15. The method of any preceding claim wherein the separation section has a length greater than 2πr.

16. The method of any of claims 3-15 wherein the separation section follows an arcuate path defined about the rotational axis.

## Patentansprüche

1. Verfahren zum Konzentrieren von pathogenen Organismen aus Wasser, enthaltend die Schritte:
a) des kontinuierlichen Zuführens von Wasser zu einem Strömungspfad, wobei der Strömungspfad einen Trennbereich enthält, der im Wesentlichen innerhalb von Ebenen angeordnet ist, die im Allgemeinen senkrecht zur Rotationsachse ausgerichtet sind, und wobei der Trennbereich eine Länge aufweist, die größer 2 r ist, wobei r das Mittel des maximalen und des minimalen radialen Abstandes des Strömungspfads von der Rotationsachse ist; und
b) des Einwirkenlassens von zentrifugalen Kräften auf den Strömungspfad, indem der Strömungspfad um die Rotationsachse gedreht wird.

2. Verfahren zum Konzentrieren von pathogenen Organismen aus Wasser, enthaltend die Schritte:
a) des kontinuierlichen Zuführens des Wassers zu einem erweiterten Strömungspfad, wobei der Strömungspfad einen Trennbereich enthält, in dem der Wasserfluss im Wesentlichen tangential zur Rotationsachse ausgerichtet ist; und
b) des Einwirkenlassens von zentrifugalen Kräften auf den Strömungspfad, indem der Strömungspfad um die Rotationsachse gedreht wird, wobei die Zentrifugalkraft im Wesentlichen senkrecht zum Trennbereich wirkt.

3. Verfahren zum Konzentrieren von pathogenen Organismen aus Wasser, enthaltend die Schritte:
a) des kontinuierlichen Zuführens von Wassers zu einem erweiterten Strömungspfad, wobei der Strömungspfad einen Trennbereich enthält, in dem das Wasser auf einem gekrümmten Weg um eine Rotationsachse fließt; und
b) des Aussetzens des Strömungspfads zu Zentrifugalkräften, indem der Strömungspfad um die Rotationsachse gedreht wird, wobei die Zentrifugalkraft im Wesentlichen senkrecht zu dem Trennbereich ausgerichtet ist.

4. Verfahren nach einem der vorhergehenden Ansprüche, wobei nach Rotieren des Strömungspfads das Wasser darin auf das Vorhandensein von pathogenen Organismen getestet wird.

5. Verfahren nach einem der vorhergehenden Ansprüche, wobei der Wasserfluss im Wesentlichen in dem gesamten Trennbereich laminar ist.

6. Verfahren nach einem der vorhergehenden Ansprüche, wobei die Oberflächen, die den Strömungspfad definieren, mit einem oberflächenaktiven Mittel beschichtet sind.

7. Verfahren nach einem der vorhergehenden Ansprüche, wobei die Zentrifugalkraft innerhalb des Trennbereichs eine relative Gravitationskraft von wenigstens 900 xg erzeugt.

8. Verfahren nach einem der vorhergehenden Ansprüche, wobei der Trennbereich die Stelle des Strömungspfads enthält, die den größten radialen Abstand von der Rotationsachse hat.

9. Verfahren nach einem der vorhergehenden Ansprüche, wobei der Strömungspfad durch eine Kanalzentrifuge (chanel centrifuge) definiert ist.

10. Verfahren nach einem der vorhergehenden Ansprüche, wobei der pathogene Organismus zu den Protozoen gehört.

11. Verfahren nach einem der vorhergehenden Ansprüche, wobei der Strömungspfad eine Wassereinlassleitung und eine Wasserauslassleitung enthält, die jeweils nahe einem Ende des Strömungspfads angeordnet sind.

12. Verfahren nach Anspruch 11, wobei die Einlass- und die Auslassleitungen einander im Strömungspfad benachbart sind, wobei dazwischen eine Barriere errichtet ist.

13. Verfahren nach Anspruch 11, wobei wenigstens entweder die Einlass- oder die Auslassleitung im Wesentlichen radial zur Rotationsachse ausgerichtet ist.

14. Verfahren nach einem der Ansprüche 2 bis 13, wobei der Trennbereich eine Länge hat, die größer 2 r ist, wobei r das Mittel des maximalen und minimalen radialen Abstands des Strömungspfads von der Rotationsachse ist.

15. Verfahren nach einem der vorhergehenden Ansprüche, wobei der Trennbereich eine Länge hat, die größer 2 π r ist.

16. Verfahren nach einem der Ansprüche 3 bis 15, wobei der Trennbereich einer gekrümmten Strecke folgt, die um die Rotationsachse definiert ist.

## Revendications

1. Procédé de concentration d'organismes pathogènes de l'eau comprenant les étapes de :
a. introduction de l'eau en continu dans un circuit d'écoulement, le circuit d'écoulement comprenant une section de séparation orientée sensiblement à l'intérieur de plans globalement perpendiculaires à l'axe de rotation, et dans lequel, la section de séparation présente une longueur supérieure à 2r, où r représente la moyenne des distances radiales maximum et minimum du circuit d'écoulement par rapport à l'axe de rotation ; et
b. soumission du circuit d'écoulement à des forces centrifuges par mise en rotation du circuit d'écoulement autour de l'axe de rotation.

2. Procédé de concentration d'organismes pathogènes de l'eau comprenant les étapes de :
a. introduction de l'eau en continu dans un circuit d'écoulement allongé, le circuit d'écoulement comprenant une section de séparation dans laquelle l'écoulement d'eau est orienté dans des directions sensiblement tangentielles par rapport à un axe de rotation ; et
b. soumission du circuit d'écoulement à des forces centrifuges par mise en rotation du circuit d'écoulement autour de l'axe de rotation, dans lequel la force centrifuge est sensiblement perpendiculaire à la section de séparation.

3. Procédé de concentration d'organismes pathogènes de l'eau comprenant les étapes de :
a. introduction de l'eau en continu dans un circuit d'écoulement allongé, le circuit d'écoulement comprenant une section de séparation dans laquelle l'eau s'écoule suivant un circuit courbe par rapport à l'axe de rotation ; et
b. soumission du circuit d'écoulement à des forces centrifuges par mise en rotation du circuit d'écoulement autour de l'axe de rotation, dans lequel la force centrifuge est sensiblement perpendiculaire à la section de séparation.

4. Procédé selon l'une quelconque des revendications précédentes, dans lequel, après avoir fait tourner le circuit d'écoulement, l'eau contenue dans celui-ci est contrôlée du point de vue de la présence d'organismes pathogènes.

5. Procédé selon l'une quelconque des revendications précédentes, dans lequel, l'écoulement d'eau est laminaire sensiblement sur toute la section de séparation.

6. Procédé selon l'une quelconque des revendications précédentes, dans lequel, les surfaces définissant le circuit d'écoulement sont revêtues par un surfactant.

7. Procédé selon l'une quelconque des revendications précédentes, dans lequel, la force centrifuge crée une force de gravitation relative à l'intérieur de la section de séparation d'au moins 900 g.

8. Procédé selon l'une quelconque des revendications précédentes, dans lequel, la section de séparation comprend le point sur le circuit d'écoulement qui est espacé avec l'étendue radiale maximum par rapport à l'axe de rotation.

9. Procédé selon l'une quelconque des revendications précédentes, dans lequel, le circuit d'écoulement est défini par un canal centrifuge.

10. Procédé selon l'une quelconque des revendications précédentes, dans lequel, les organismes pathogènes sont des protozoaires.

11. Procédé selon l'une quelconque des revendications précédentes, dans lequel, le circuit d'écoulement comprend une conduite d'entrée d'eau et une conduite de sortie d'eau, chacune étant située à proximité d'une extrémité du circuit d'écoulement.

12. Procédé selon la revendication 11, dans lequel les conduites d'entrée et de sortie sont adjacentes l'une à l'autre sur le circuit d'écoulement, une barrière étant placée entre elles.

13. Procédé selon la revendication 11, dans lequel au moins une des conduites d'entrée et de sortie est orientée sensiblement radialement par rapport à l'axe de rotation.

14. Procédé selon l'une quelconque des revendications 2 à 13, dans lequel la section de séparation présente une longueur supérieure à 2r, où r représente la moyenne des distances radiales maximum et minimum du circuit d'écoulement par rapport à l'axe de rotation.

15. Procédé selon l'une quelconque des revendications précédentes, dans lequel, la section de séparation présente une longueur supérieure à 2πr.

16. Procédé selon l'une quelconque des revendications 3 à 15, dans lequel la section de séparation suit un circuit courbe défini autour de l'axe de rotation.
